# EUROPÄISCHE PATENTANMELDUNG

(11) **EP 0 759 316 A1**
(43) Veröffentlichungstag der Anmeldung: **26.02.1997**
(21) Anmeldenummer: 96113183.6
(22) Anmeldetag: 16.08.1996
(51) Int. Cl.: B01D 3/16

(54) **Vorrichtung und Verfahren zum Stoffaustausch in Bodenkolonnen**

(30) Priorität: 17.08.1995 DE 19530291
(71) Anmelder: BASF AKTIENGESELLSCHAFT, 67056 Ludwigshafen (DE)
(72) Erfinder: Altinger, Gerhard, 67346 Speyer (DE); Egly, Horst, 67459 Böhl-Iggelheim (DE); Kaiser, Rudolf, 67069 Ludwigshafen (DE); Thiessen, Fritz, Dr., 67071 Ludwigshafen (DE)
(74) Vertreter: Isenbruck, Günter, Dr.

(57) **Zusammenfassung**

Kolonnenboden für eine, insbesondere mit einem Auflagering am Umfang versehene, Stoffaustauschkolonne, der Stützelemente und nebeneinander an den Stützelementen angeordnete Bodenplatten mit Durchlaßöffnungen, insbesondere Löchern, Ventilen oder Glocken, aufweist, wobei die Mehrzahl, vorzugsweise die Gesamtzahl, der Bodenplatten mit den Stützelementen bzw. dem Auflagering, insbesondere in diskreten Flächenelementen, flüssigkeitsdicht verbunden, vorzugsweise verschweißt ist; die Mehrzahl, vorzugsweise die Gesamtzahl, der Stützelemente miteinander und mit der Kolonnenwand bzw. dem Auflagering, insbesondere in diskreten Flächenelementen, flüssigkeitsdicht verbunden, vorzugsweise verschweißt ist; und im übrigen die Mehrzahl, vorzugsweise die Gesamtzahl, der benachbarten Bodenplatten und Stützelemente voneinander und von der Kolonnenwand bzw. dem Auflagering durch Spalte mit einer Breite von 1 mm bis 15 mm, vorzugsweise von 1 mm bis 7 mm, getrennt ist.

## Beschreibung

Die Erfindung betrifft eine Vorrichtung und ein Verfahren zum Stoffaustausch zwischen Flüssigkeiten und Gasen in Bodenkolonnen. Insbesondere wird eine spezielle Ausgestaltung von Kolonnenböden vorgeschlagen.

Bodenkolonnen werden heute für eine Vielzahl von Stoffaustauschprozessen zwischen Flüssigkeiten und Gasen verwendet. Dabei überströmt die Flüssigkeit den Kolonnenboden, der eine Vielzahl von Öffnungen aufweist. Durch diese Öffnungen wird Gas in die Flüssigkeit eingespeist, so daß der Stoffaustauschvorgang stattfinden kann. Die Flüssigkeit wird durch die Öffnungen oder durch spezielle Ableiteinrichtungen von Boden zu Boden weitergeleitet.

Für die Bodenöffnungen ist eine Vielzahl von Gestaltungsmöglichkeiten bekannt. Es können plane Öffnungen vorgesehen sein (Siebböden; Dualflowböden), die Öffnungen können mit Ventilen versehen sein (Ventilböden) und die Öffnungen können auch mit Glocken gegen die Flüssigkeit abgeschirmt sein (Glockenböden). Bekannt sind auch höher integrierte Bodengestaltungen wie Tunnel- und Zentrifügalböden, bei denen immer mehrere Öffnungen zu einer Gruppe von Gaseinlässen zusammengefaßt sind und bei denen die Richtung des Flüssigkeitsstromes durch den Impuls des ausströmenden Gases gesteuert wird.

Bei den heute verwendeten Bodenkolonnen mit Durchmessern von mehreren Metern sind die Böden aus mehreren Planen zusammengesetzt, die auf Trägern bzw. auf Stützelementen wie einem Randring oder einzelnen Auflagestücken an der Kolonnenwand gelagert oder befestigt sind.

Die an Trägern befestigten Bodenplatten werden dabei bündig aneinandergelegt und mit Hilfe von Klammern oder Klemmleisten miteinander verschraubt. In anderen Ausführungsformen werden die Bodenplanen überlappend angeordnet oder es werden Keile von oben in die Spalte zwischen den Bodenplanen eingeschoben. Neben einer Erhöhung der Festigkeit der Bodenstruktur sollen all diese Maßnahmen den Durchtritt von Flüssigkeit zwischen den Bodenplatten statt an den vorgesehenen Flüssigkeitsabläufen verhindern. Selbst bei Wellsieb- oder Turbogritböden, bei denen die Flüssigkeit durch Gaszuleitungsöffnungen abläuft, ist dieser zusätzliche unkontrollierte Flüssigkeitsablauf unerwünscht.

Bei allen genannten Konstruktionen entstehen sehr feine Durchlässe, insbesondere Kapillarspalte (Haarspalte), an den Verbindungslinien von Bodenplatten miteinander oder mit Stützelementen (Trägern) oder an Schraubverbindungen der Bodenteile. In derartigen feinen Durchlässen ist die Verweilzeit der Flüssigkeit um ein Vielfaches höher als die durchschnittliche Verweilzeit eines Flüssigkeitsvolumens auf einem Kolonnenboden. Dies führt bei bestimmten Stoffen zu Polymerisation, Koagulation, Verbackung oder sonstigen unerwünschten physikalischen oder chemischen Stoffveränderungen. Häufig sind die Haarspalte nur der Startpunkt für derartige Reaktionen, die sich dann auf dem Kolonnenboden fortsetzen.

Die Haarspalte sind auch neuralgische Punkte hinsichtlich der Sammlung unerwünschter Substanzen (Schmutz), insbesondere wegen der an diesen Stellen auftretenden Reinigungsschwierigkeiten. Diese konstruktiv bedingten Haarspalte sind deshalb auch Ausgangspunkt und Ursache für eine Reihe von Verschmutzungs-, Verbackungs- und Polymerisationsproblemen.

Es ist mit vertretbarem Aufwand technisch bisher nicht möglich, die Bildung von feinen Durchlässen, insbesondere von Kapillarspalten, zwischen den Bauteilen eines Kolonnenbodens zu verhindern. Weder genauere Passungen noch höhere Anpreßdrücke durch eine festere Verschraubung oder Vernietung, noch die Einfügung von Dichtmassen führen zu einer Lösung dieses Problems. Letztere sind bei einem Kolonnenboden insbesondere deshalb problematisch, weil die infolge der Gasdurchströmung auftretenden Schwingungen der Bodenkonstruktion die Haftung von Dichtmassen in Fugen stark beeinträchtigt.

Aufgabe der vorliegenden Erfindung ist deshalb die Bereitstellung eines Kolonnenbodens zum Einbau in eine Stoffaustauschkolonne und eines Verfahrens zum Stoffaustausch in einer Bodenkolonne, bei dem die Polymerisation, Koagulation, Verbackung, Verschmutzung und sonstige physikalische oder chemische Stoffveränderung in feinen Durchlässen zwischen den Bauteilen des Kolonnenbodens vermieden werden.

Diese Aufgabe wird durch die in den Ansprüchen beschriebenen Kolonnenböden gelöst. Ein erfindungsgemäßer Kolonnenboden für eine, insbesondere mit einem Auflagering am Umfang versehene, Stoffaustauschkolonne besteht aus Stützelementen und nebeneinander an den Stützelementen angeordneten Bodenplatten mit Durchlaßöffnungen, insbesondere Löchern, Ventilen oder Glocken. Der erfindungsgemäße Kolonnenboden ist dadurch gekennzeichnet, daß die Mehrzahl, vorzugsweise die Gesamtzahl, der Bodenplatten mit den Stützelementen bzw. dem Auflagering, insbesondere in diskreten Flächenelementen, flüssigkeitsdicht verbunden, vorzugsweise verschweißt ist; die Mehrzahl, vorzugsweise die Gesamtzahl, der Stützelemente miteinander und mit der Kolonnenwand bzw. dem Auflagering, insbesondere in diskreten Flächenelementen, flüssigkeitsdicht verbunden, vorzugsweise verschweißt ist; und im übrigen die Mehrzahl, vorzugsweise die Gesamtzahl, der benachbarten Bodenplatten und Stützelemente voneinander und von der Kolonnenwand bzw. dem Auflagering durch Spalte mit einer Breite von 1 mm bis 15 mm, vorzugsweise von 1 mm bis 7 mm, getrennt sind. Somit wird einer Bildung von unerwünschten Spalten dadurch entgegengewirkt, daß einerseits die Spalte groß genug gemacht werden, um die beschriebenen nachteiligen Reaktionen zu vermeiden, und andererseits die Abdichtung kleiner Flächen so gut erfolgt, daß Spaltbildung mit vertretbarem technischen Aufwand vermieden werden kann. Besonders vorteilhaft ist dabei die Herstellung kleinflächiger Schweißverbindungen zwischen den zu verbindenden Bauteilen, da dadurch in vorteilhafter Weise eine flüssigkeitsdichte Verbindung geschaffen werden kann.

Diese erfindungsgemäße Gestaltung sollte möglichst für alle Kontaktflächen von Bodenplatten, Stützelementen und der Kolonnenwand bzw. einem Auflagering an der Kolonnenwand gewählt werden. Natürlich führt bereits die Gestaltung der überwiegenden Zahl der Kontaktflächen zu einem erfindungsgemäßen positiven Ergebnis. Es kann aber aus anderen Gründen zweckmäßig sein, an bestimmten Stellen, z.B. an Durchstiegen, andere Verbindungsarten, z.B. eine Verschraubung, zu wählen.

Vorzugsweise ist die Mehrzahl, insbesondere die Gesamtzahl, der Bodenplatten mit viaduktartigen Stützelementen verbunden, insbesondere verschweißt. Dabei sind die Viaduktbögen zu den Bodenplatten hin offen, so daß es nicht zu einer Aufteilung des Stoffaustausches in Bodensegmente kommt, sondern die Flüssigkeit möglichst frei auf dem Kolonnenboden fließen kann. Die Verwendung von an den Füßen verschweißten Viaduktbögen führt überdies zu einer leichten Stütz- bzw. Tragekonstruktion.

Bevorzugt ist ein Kolonnenboden, dessen Flächenanteil der in der Bodenebene bestehenden Spalte an dem Gesamtöffnungsverhältnis höchstens 15% beträgt. Das Gesamtöffnungsverhältnis ist definiert als Anteil der gasdurchlässigen Fläche (Spalte, Durchlaßöffnungen) in der Bodenebene an der Gesamtfläche der Bodenebene (Kolonnenquerschnitt). Dabei wird die Fläche von Ablaufschächten nicht mitgerechnet, da dort kein Stoffaustausch stattfindet. Besonders bevorzugt ist ein Kolonnenboden, der als Dualflowboden ausgelegt und in der Stoffaustauschkolonne angeordnet ist. Besonders vorteilhaft ist dabei die Kombination mit den oben beschriebenen Viaduktbögen.

Die erfindungsgemäße Aufgabe wird auch durch das in den Ansprüchen beschriebene Verfahren zum Stoffaustausch in einer Bodenkolonne gelöst. Dabei wird Flüssigkeit auf einem Kolonnenboden, der Stützelemente und nebeneinander an den Stützelementen angeordnete Bodenplatten mit Durchlaßöffnungen, insbesondere Löchern, Ventilen oder Glocken, aufweist, in der Bodenebene bewegt und der Flüssigkeit Gas durch die Durchlaßöffnungen zugeführt, wobei die Mehrzahl, vorzugsweise die Gesamtzahl, der Bodenplatten mit den Stützelementen bzw. dem Auflagering, insbesondere in diskreten Flächenelementen, flüssigkeitsdicht verbunden, vorzugsweise verschweißt werden; die Mehrzahl, vorzugsweise die Gesamtzahl, der Stützelemente miteinander und mit der Kolonnenwand bzw. dem Auflagering, insbesondere in diskreten Flächenelementen, flüssigkeitsdicht verbunden, vorzugsweise verschweißt werden; und im übrigen die Mehrzahl, vorzugsweise die Gesamtzahl, der benachbarten Bodenplatten und Stützelemente voneinander und von der Kolonnenwand bzw. dem Auflagering durch Spalte mit einer Breite von 1 mm bis 15 mm, vorzugsweise von 1 mm bis 7 mm, getrennt werden.

Bevorzugt ist ein Verfahren, bei dem die Mehrzahl, vorzugsweise die Gesamtzahl der Bodenplatten, mit viaduktartigen Stützelementen verbunden, insbesondere verschweißt wird, wobei die Viaduktbögen zu den Bodenplatten hin offen sind.

Bevorzugt ist auch ein Verfahren, bei dem die Spalte in der Bodenebene derart angeordnet und bemessen sind, daß ihr Flächenanteil an dem Gesamtöffnungsverhältnis höchstens 15% beträgt.

Weiter bevorzugt ist ein Verfahren, bei dem Flüssigkeit und Gas nach dem Dualflowprinzip in einem Sprudelbett zusammengeführt werden.

Erfindungsgemäß wird auch eine Stoffaustauschkolonne bereitgestellt, die mindestens einen in den Ansprüchen beschriebenen Kolonnenboden aufweist.

Entsprechend der Aufgabe der vorliegenden Erfindung wird auch ein Verfahren zur Herstellung eines Kolonnenbodens für eine Stoffaustauschkolonne bereitgestellt, wobei Bodenplatten mit Durchlaßöffnungen, insbesondere Löchern, Ventilen oder Glocken, nebeneinander an Stützelementen bzw. dem Auflagering angeordnet werden, und das dadurch gekennzeichnet ist, daß die Mehrzahl, vorzugsweise die Gesamtzahl, der Bodenplatten mit den Stützelementen bzw. dem Auflagering, insbesondere in diskreten Flächenelementen, flüssigkeitsdicht verbunden, vorzugsweise verschweißt werden, und die Mehrzahl, vorzugsweise die Gesamtzahl, der Stützelemente miteinander und mit der Kolonnenwand bzw. dem Auflagering, insbesondere in diskreten Flächenelementen, flüssigkeitsdicht verbunden, vorzugsweise verschweißt werden. Dabei werden im übrigen die Mehrzahl, vorzugsweise die Gesamtzahl, der benachbarten Bodenplatten und Stützelemente voneinander und von der Kolonnenwand bzw. dem Auflagering durch Spalte mit einer Breite von 1 mm bis 15 mm, vorzugsweise von 1 mm bis 7 mm, getrennt.

Bevorzugt ist ein Herstellungsverfahren, bei dem die Mehrzahl, vorzugsweise die Gesamtzahl, der Bodenplatten mit viaduktartigen Stützelementen verbunden, insbesondere verschweißt wird, wobei die Viaduktbögen zu den Bodenplatten hin offen sind.

Weiter bevorzugt ist ein Verfahren, bei dem die Bodenplatten mit Durchlaßöffnungen derart gestaltet und in der Bodenkolonne angeordnet werden, daß der Flächenanteil der in der Bodenebene bestehenden Spalte an dem Gesamtöffnungsverhältnis höchstens 15% beträgt.

Bevorzugt werden derartige Kolonnenböden in der Stoffaustauschkolonne als Dualflowböden ausgelegt und angeordnet.

Bei den in den Ansprüchen beschriebenen Bodenkolonnen und Verfahren soll das Hindurchtreten von Flüssigkeit durch Kapillarspalte, d.h. Spalte mit einer Breite kleiner 0,5 mm, verhindert werden. Dazu ist es notwendig, Spalte an Verbindungen zwischen Platten und Halteeinrichtungen entweder völlig zu vermeiden oder so groß zu machen, daß die Flüssigkeit weitgehend ungehindert hindurchtreten kann. Versuche haben gezeigt, daß das fluiddynamische Verhalten der erfindungsgemäßen Bodenkonstruktion im Vergleich zur konventionellen Bodengestaltung nur geringfügige Nachteile aufweist. Minimal sind diese Nachteile insbesondere bei der bereits beschriebenen viaduktartigen Gestaltung der Stützelemente.

Die Erfindung wird im folgenden anhand dreier Figuren erläutert:
- Fig. 1:: Prinzipskizze eines erfindungsgemäßen Kolonnenbodens;
- Fig. 2:: Druckverlust eines erfindungsgemäßen Kolonnenbodens (Dualflowboden) in Abhängigkeit von der relativen Spaltfläche;
- Fig. 3:: Druckverlust eines erfindungsgemäßen Kolonnenbodens in Abhängigkeit vom Durchmesser des Wandringspaltes.

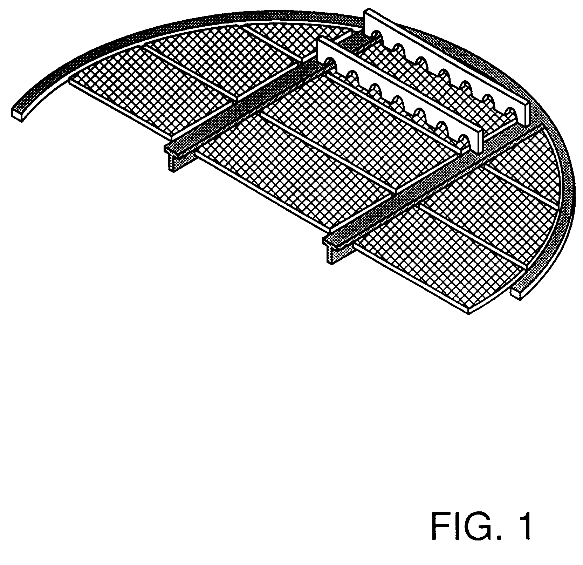

In Fig. 1 wird ein erfindungsgemäßer Kolonnenboden dargestellt. Viaduktähnliche Stützelemente, die jeweils mit den Bodenplatten und den anderen Stützelementen (Trägern) und mit dem an der Kolonnenwand angebrachten Auflagering verschweißt sind, halten die Bodenplatten. Zwischen den Bodenplatten untereinander, zwischen den Bodenplatten und den Stützelementen sowie zwischen den Stützelementen bzw. dem Auflagering untereinander befinden sich im übrigen Spalte von etwa 5 mm Breite. Je nach Kolonnendurchmesser ergeben sich fertigungsbedingt mehr oder weniger große Streuungen der Spaltbreite. Um Kapillarspalte zu vermeiden, darf die Spaltbreite 1 mm nicht unterschreiten.

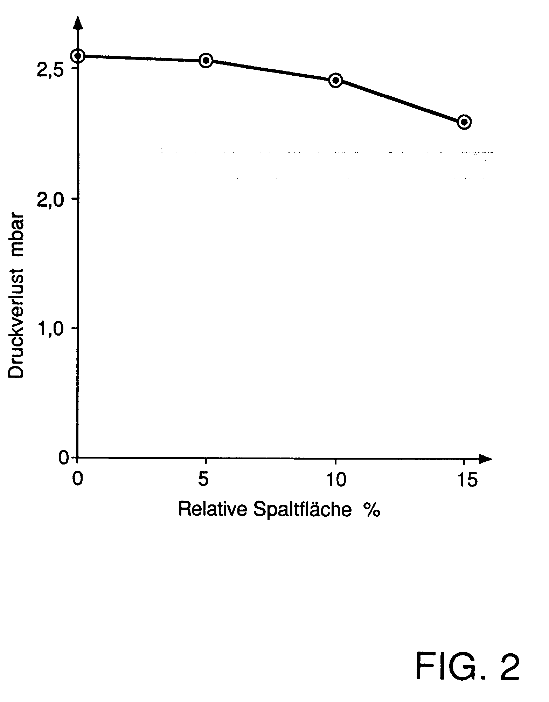

Fig. 2 stellt den Druckverlust eines Dualflowbodens in mbar in Abhängigkeit von der relativen Spaltfläche dar. Als relative Spaltfläche wird das Verhältnis der Spaltfläche in der Bodenebene im Vergleich zur Gesamtfläche in der Bodenebene, durch die Flüssigkeit hindurchtreten kann (Spalte, Löcher, etc.), verstanden.

Die Meßkurven wurden an Dualflowböden in einer Kolonne mit 2500 mm Durchmesser aufgenommen. Der Lochdurchmesser betrug 14 mm, die Spaltbreite 5 mm, die Bodendicke 5 mm, und das Gesamtöffnungsverhältnis betrug durch Variation der Lochzahl stets 13,2%. An der Kolonnenwand befand sich ein Auflagering von 40 mm Breite. Drei Dualflowböden waren jeweils in einem Abstand von 400 mm zueinander angebracht. Zusätzlich wurde noch ein Verteilerboden eingebaut. Die Messungen wurden mit Wasser und Luft am mittleren Boden durchgeführt. Dabei wurde Wasser in einer Menge von 3,6m³/m²h und Luft jeweils in einer Menge entsprechend einer Strömungsgeschwindigkeit von 1,1 m/s zugeführt.

Es ergab sich, daß der Druckverlust mit steigender relativer Spaltfläche abnahm. Es sollten also nur soviele Spalte vorgesehen werden, wie für die einwandfreie Funktion im Sinne dieser Erfindung, d.h. zur Vermeidung von Koagulation und Verschmutzung, notwendig sind.

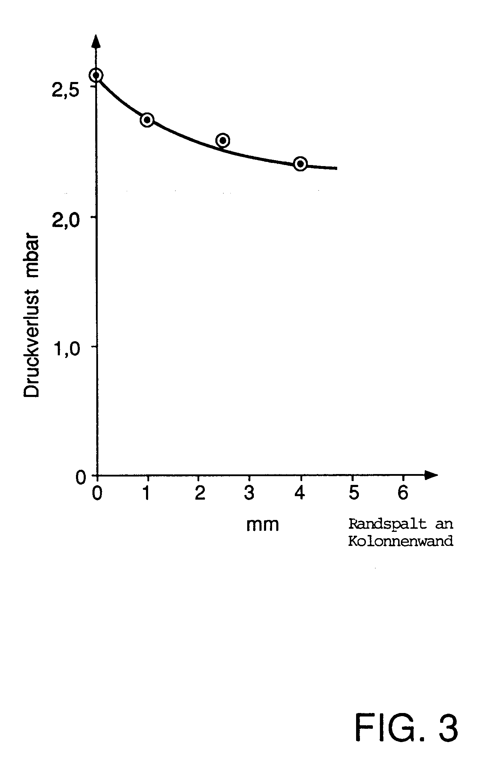

Fig. 3 zeigt die Abhängigkeit des Druckverlustes in mbar von der Breite des Ringspaltes entlang der Kolonnenwand.

Statt Spalten zwischen den einzelnen Bauteilen wurde hier für Demonstrationszwecke ein einziger Randspalt an der Kolonnenwand vorgesehen, der nur durch wenige notwendige Halterungen für die Kolonnenböden unterbrochen war. Der Kolonnendurchmesser betrug 2500 mm, der Lochdurchmesser 14 mm und die Bodendicke 5 mm. Die Wasser- und Luftzufuhr sowie die Messung des Druckverlustes entsprach dem unter Fig. 2 beschriebenen Vorgehen. Das Gesamtöffnungsverhältnis wurde wiederum durch Variation der Lochzahl auf 13,2% konstant gehalten.

Es ergab sich, daß der Druckverlust mit steigender Randspalt-Breite stark abnahm. Es ist deshalb vorteilhaft, einen Randspalt an der Kolonnenwand so klein zu halten, daß die erfindungsgemäße Funktion gerade ausgeführt wird. Im Interesse eines effektiven Stoffaustausches wird ein Ringspalt an der Kolonnenwand überhaupt vermieden.

### BEISPIEL

Ein typisches Beispiel für die Anwendung eines erfindungsgemäß konstruierten Kolonnenbodens ist die Trennung von Acrylsäure von Nebenprodukten. Hier bildet sich in technischen Destillationskolonnen trotz des Zusatzes von Stabilisatoren Polymerisat auf den Destillierböden.

Dieser Vorgang beruht vor allem auf der langen Verweilzeit der Acrylsäure in feinen, insbesondere in Kapillarspalten. Dadurch wird die Entstehung und das Wachstum von Polymerisat und die Ablagerung von Schmutz begünstigt.

Dies kann anhand eines einfachen Versuches illustriert werden. Dabei wird Acrylsäure einmal über eine Schraube, an der eine Unterlegscheibe mit einer Mutter eingeklemmt ist, und parallel dazu über eine plane Blechplatte laufen gelassen. Nach 5 bis 8 Tagen tritt bei Schraube und Mutter deutlich sichtbar Polymerisat auf, während an dem planen Blech derartige Ablagerungen nicht zu erkennen sind.

Die im Versuch verwendete Acrylsäure war mit 200 ppm Phenothiazin stabilisiert. Es handelte sich um eine Roh-Acrylsäure, die durch katalytische Gasphasenoxidation von Acrolein gemäß Beispiel B1 der DE 43 02 991 und durch anschließende Aufarbeitung der Reaktionsgase gemäß Beispiel B1 der DE 21 36 396 erhalten worden war.

## Patentansprüche

1. Kolonnenboden für eine, insbesondere mit einem Auflagering am Umfang versehene, Stoffaustauschkolonne, der Stützelemente und nebeneinander an den Stützelementen angeordnete Bodenplatten mit Durchlaßöffnungen, insbesondere Löchern, Ventilen oder Glocken, aufweist, dadurch gekennzeichnet, daß
die Mehrzahl, vorzugsweise die Gesamtzahl, der Bodenplatten mit den Stützelementen bzw. dem Auflagering, insbesondere in diskreten Flächenelementen, flüssigkeitsdicht verbunden, vorzugsweise verschweißt ist,
die Mehrzahl, vorzugsweise die Gesamtzahl, der Stützelemente miteinander und mit der Kolonnenwand bzw. dem Auflagering, insbesondere in diskreten Flächenelementen, flüssigkeitsdicht verbunden, vorzugsweise verschweißt ist, und
im übrigen die Mehrzahl, vorzugsweise die Gesamtzahl, der benachbarten Bodenplatten und Stützelemente voneinander und von der Kolonnenwand bzw. dem Auflagering durch Spalte mit einer Breite von 1 mm bis 15 mm, vorzugsweise von 1 mm bis 7 mm, getrennt ist.

2. Kolonnenboden nach Anspruch 1, dadurch gekennzeichnet, daß die Mehrzahl, vorzugsweise die Gesamtzahl, der Bodenplatten mit viaduktartigen Stützelementen verbunden, insbesondere verschweißt ist, wobei die Viaduktbögen zu den Bodenplatten hin offen sind.

3. Kolonnenboden nach Anspruch 1 oder 2, dadurch gekennzeichnet, daß der Flächenanteil der in der Bodenebene bestehenden Spalte an dem Gesamtöffnungsverhältnis höchstens 15% beträgt.

4. Kolonnenboden nach einem der Ansprüche 1 bis 3, dadurch gekennzeichnet, daß er als Dualflowboden ausgelegt und in der Stoffaustauschkolonne angeordnet ist.

5. Verfahren zum Stoffaustausch in einer, insbesondere mit einem Auflagering am Umfang versehenen, Stoffaustauschkolonne, wobei Flüssigkeit auf einem Kolonnenboden, der Stützelemente und nebeneinander an den Stützelementen angeordnete Bodenplatten mit Durchlaßöffnungen, insbesondere Löchern, Ventilen oder Glocken, aufweist, in der Bodenebene bewegt wird und der Flüssigkeit Gas durch die Durchlaßöffnungen zugeführt wird, dadurch gekennzeichnet, daß
die Mehrzahl, vorzugsweise die Gesamtzahl, der Bodenplatten mit den Stützelementen bzw. dem Auflagering, insbesondere in diskreten Flächenelementen, flüssigkeitsdicht verbunden, vorzugsweise verschweißt wird,
die Mehrzahl, vorzugsweise die Gesamtzahl, der Stützelemente miteinander und mit der Kolonnenwand bzw. dem Auflagering, insbesondere in diskreten Flächenelementen, flüssigkeitsdicht verbunden, vorzugsweise verschweißt wird, und
im übrigen die Mehrzahl, vorzugsweise die Gesamtzahl, der benachbarten Bodenplatten und Stützelemente voneinander und von der Kolonnenwand bzw. dem Auflagering durch Spalte mit einer Breite von 1 mm bis 15 mm, vorzugsweise von 1 mm bis 7 mm, getrennt wird.

6. Verfahren nach Anspruch 5, dadurch gekennzeichnet, daß die Mehrzahl, vorzugsweise die Gesamtzahl, der Bodenplatten mit viaduktartigen Stützelementen verbunden, insbesondere verschweißt wird, wobei die Viaduktbögen zu den Bodenplatten hin offen sind,
daß die Spalte in der Bodenebene derart angeordnet und bemessen sind, daß ihr Flächenanteil an dem Gesamtöffnungsverhältnis höchstens 15% beträgt, und
daß Flüssigkeit und Gas nach dem Dualflowprinzip in einem Sprudelbett zusammengeführt werden.

7. Stoffaustauschkolonne mit mindestens einem Kolonnenboden nach einem der Ansprüche 1 bis 4.

8. Verfahren zur Herstellung eines Kolonnenbodens für eine, insbesondere mit einem Auflagering am Umfang versehene, Stoffaustauschkolonne, wobei Bodenplatten mit Durchlaßöffnungen, insbesondere Löchern, Ventilen oder Glocken, nebeneinander an Stützelementen bzw. dem Auflagering angeordnet werden, dadurch gekennzeichnet, daß
die Mehrzahl, vorzugsweise die Gesamtzahl, der Bodenplatten mit den Stützelementen bzw. dem Auflagering, insbesondere in diskreten Flächenelementen, flüssigkeitsdicht verbunden, vorzugsweise verschweißt wird,
die Mehrzahl, vorzugsweise die Gesamtzahl, der Stützelemente miteinander und mit der Kolonnenwand bzw. dem Auflagering, insbesondere in diskreten Flächenelementen, flüssigkeitsdicht verbunden, vorzugsweise verschweißt wird, und
im übrigen die Mehrzahl, vorzugsweise die Gesamtzahl, der benachbarten Bodenplatten und Stützelemente voneinander und von der Kolonnenwand bzw. dem Auflagering durch Spalte mit einer Breite von 1 mm bis 15 mm, vorzugsweise von 1 mm bis 7 mm, getrennt angeordnet wird.

9. Verfahren nach Anspruch 8, dadurch gekennzeichnet, daß die Mehrzahl, vorzugsweise die Gesamtzahl, der Bodenplatten mit viaduktartigen Stützelementen verbunden, insbesondere verschweißt wird, wobei die Viaduktbögen zu den Bodenplatten hin offen sind.

10. Verfahren nach einem der Ansprüche 8 oder 9, dadurch gekennzeichnet, daß die Bodenplatten mit Durchlaßöffnungen derart gestaltet und angeordnet werden, daß der Flächenanteil der in der Bodenebene bestehenden Spalte an dem Gesamtöffnungsverhältnis höchstens 15% beträgt, und daß der Kolonnenboden als Dualflowboden ausgelegt und in der Stoffaustauschkolonne angeordnet wird.
